(19) Europäisches Patentamt
European Patent Office
Office européen des brevets

(11) Veröffentlichungsnummer: **0 012 278**
**B1**

(12) # EUROPÄISCHE PATENTSCHRIFT

(45) Veröffentlichungstag der Patentschrift: **19.05.82**

(21) Anmeldenummer: **79104762.4**

(22) Anmeldetag: **29.11.79**

(51) Int. Cl.³: **C 07 D 211/46,**
C 07 H 13/12, C 12 P 19/26
//(C12P19/26, C12R1/00)

(54) **Herstellung von N-substituierten Derivaten des 1-Desoxy-nojirimycins.**

(30) Priorität: **12.12.78 DE 2853573**

(43) Veröffentlichungstag der Anmeldung:
**25.06.80 Patentblatt 80/13**

(45) Bekanntmachung des Hinweises auf die Patenterteilung:
**19.05.82 Patentblatt 82/20**

(84) Benannte Vertragsstaaten:
**CH DE FR GB IT NL**

(56) Entgegenhaltungen:
**DE - A - 2 846 118**
**EP - A - 0 007 040**

**Chem Ber 100, 802—815 (1967), Chem. Pharm. Bull. 16 (12), 2477—2481 (1968), Can. J. Chem. 39, 2400—2410 (1961), Adv. Carbolych. Chem. 23, 115 (1968), Jap. Patentanm. 55105—666 und 55105—667**

**TETRAHEDRON, Band 23, 1968**
**Pergamon Press, GB.**
**S. INOUYE et al.: "Structure and synthesis of nojirimycin", Seiten 2125—2144**

(73) Patentinhaber: **BAYER AG**
**Zentralbereich Patente, Marken und Lizenzen**
**D-5090 Leverkusen 1, Bayerwerk (DE)**

(72) Erfinder: **Kinast, Günther, Dr.**
**Am Eckbusch 35/33**
**D-5600 Wuppertal 1 (DE)**
Erfinder: **Schedel, Michael, Dr.**
**Sillerstrasse 49**
**D-5600 Wuppertal 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 012 278 B1

# Herstellung von N-substituierten Derivaten des 1-Desoxynojirimycins

Die vorliegende Erfindung betrifft ein neues Verfahren zur Herstellung der Verbindungen der Formel

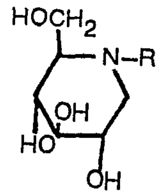

(I)

worin R Wasserstoff, gegebenenfalls durch OH, $C_1$-$C_4$-Alkoxy oder Di-$C_1$-$C_4$-alkylamino substituiertes $C_1$-$C_{10}$-Alkyl bedeutet.

Es ist bereits bekannt geworden, daß die unter dem Namen 1-Desoxynojirimycin bekannte Verbindung der Formel (I; R=H) entweder durch Extraktion von Pflanzen der Moros-Arten gemäß deutscher Offenlegungsschrift 2 656 602 oder mikrobiologisch mit Hilfe von Organismen der Familie Bacillaceae, insbesondere des Stammes DSM 7 gemäß deutscher Offenlegungsschrift 2 658 563 erhalten werden kann. Die Verbindungen der Formel (I) können als Mittel gegen Diabetes, Hyperlipoproteinämie und Adipositas verwendet werden.

Außerdem ist bereits bekannt geworden, daß man 1-Desoxynojirimycin durch Hydrierung der wenig stabilen freien Basen (II) oder (III)

(II) 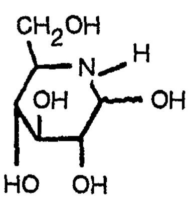 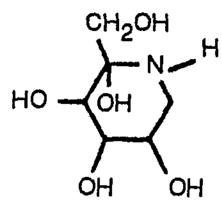 (III)

nach H. Saki und E. Ohki, Chem. Pharm.

Bull. *16*, 2477 bis 2481 (1968) und H. Paulsen, I. Sangster und H. Heyns, Chem. Ber. *100*, 802 bis 815 (1967) herstellen kann.

Die dabei als Ausgangsprodukt verwandte Verbindung (II) wird hergestellt, indem man entweder 5-Amino-5-desoxy-1.2-O-isopropyliden-$\alpha$-D-glucofuranose (IV) durch 60-stündiges Einleiten von Schwefeldioxid in ein stabiles Bisulfit-Addukt (V) überführt, welches dann durch Behandlung mit Bariumhydroxid II ergibt,

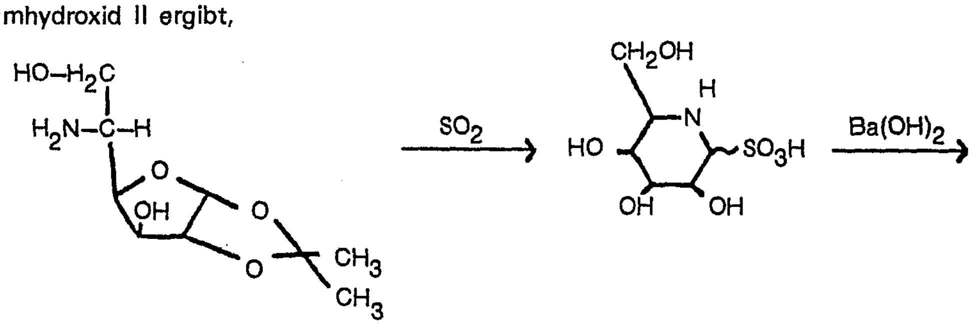


(II)

(IV) (V).

(Deutsche Auslegeschrift 1 768 044)

oder indem man die Verbindung IV in das trifluoracetylierte Derivat VI überführt, welches durch Verkochen mit verdünnter Salzsäure das Trifluoracetylderivat VII ergibt. Durch anschließende Abspaltung der Trifluoracetylgruppen erhält man (II)

(IV)

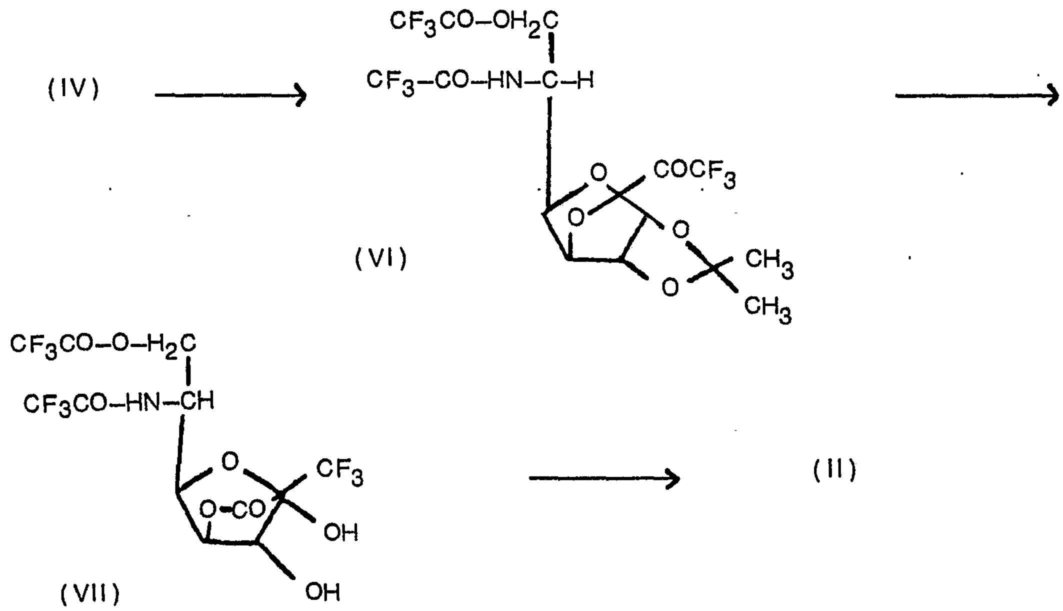

(VI)

(VII)

(II)

oder man stellt die Verbindung (II) ausgehend von Glucose mikrobiologisch her (US—PS 3 998 698).

Die Verbindung (III) gewinnt man aus 6-Amino-2.3-O-isopropyliden-6-desoxy-$\alpha$-L-sorbofuranose VIII durch Spaltung und anschließende Freisetzung des erhaltenen Hydrochlorides IX durch Chromatographie über einen Anionenaustauscher.

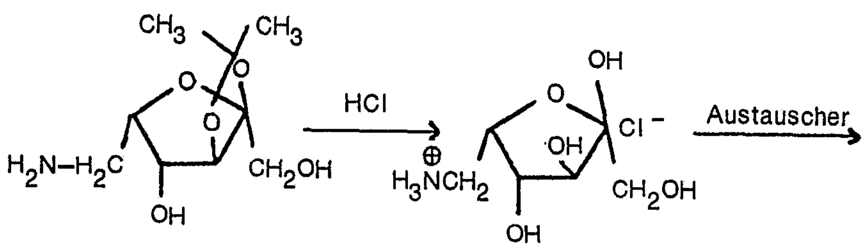


(III)

VIII IX

Die hier aufgeführten Verfahren zur Herstellung von 1-Desoxynojirimycin verlaufen alle über mehrere zeitraubende Stufen, wobei das Durchlaufen der instabilen Verbindungen II und III als Zwischenprodukte zwangsläufig zu Nebenprodukten führen muß.

Außerdem erfordern die bisher bekannten Verfahren zur Herstellung der Verbindung I (R=H) in jedem Fall aufwendige Reinigungsschritte wie Extraktion, Säulenchromatographie oder Chromatographie an Austauschern, die zum Teil auch dadurch notwendig werden, daß die Hydrierung der freien Basen nicht stereospezifisch verläuft.

Es wurde nun überraschend gefunden, daß man die Verbindungen I in ausgezeichneten Ausbeuten erhält, wenn man Verbindungen der allgemeinen Formel (X)

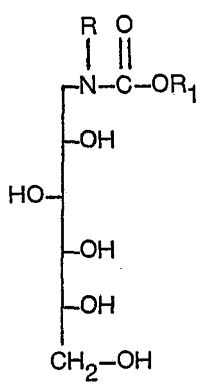

(X)

worin R die bereits genannte Bedeutung hat und $R_1$ gegebenenfalls durch Chlor, Brom, Nitro, Methyl, oder Methoxy substituiertes Benzyl oder Allyl bedeutet, in an sich bekannter Weise mit aeroben Mikroorganismen oder Extrakten daraus in geeigneten Medien, die assimilierbare Kohlenstoff- und Stickstoffquellen sowie Mineralsalze enthalten und bei pH-Werten zwischen 2 und 10 und bei Temperaturen zwischen 20 und 45°C zu Verbindungen der Formel

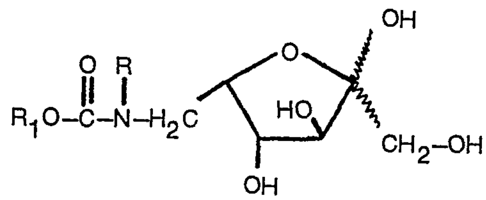

worin R und $R_1$ die bereits genannte Bedeutung haben, umsetzt wobei als aerobe Mikroorganismer solche eingesetzt werden, die in einem entsprechenden Nährmedium enthaltend eine Verbindung der Formel

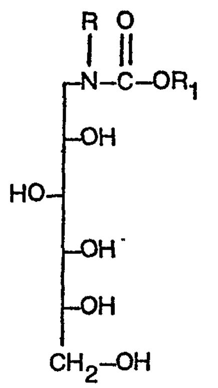

worin R und $R_1$ die angegebene Bedeutung besitzen, eine Verbindung der Formel

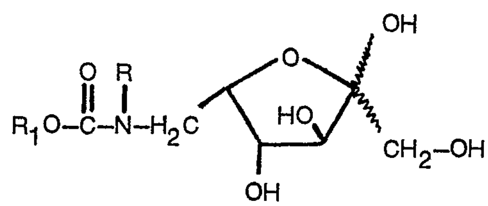

anhäufen, und

b) diese Verbindungen bei einem pH-Wert zwischen 0 und 7 und in einem geeigneten Lösungsmittel unter Erhalt der Konfiguration katalytisch hydriert.

6-Amino-6-desoxy-L-sorbose (Formel XI; $R = R_1 = H$) wurde bisher in einer vielstufigen Synthese auf rein chemischem Wege aus Sorbose hergestellt. (H. Paulsen, I. Sangster und K. Heins, Chem. Ber. *100*, 802 bis 815 (1967)).

Das erfindungsgemäße Verfahren in seiner Gesamtheit ergibt sich nicht in naheliegender Weise aus dem Stand der Technik. Nach diesem Verfahren können nämlich die Verbindungen der allgemeinen Formel I, die als solche bekannt ist, in nur vier Syntheseschritten, ausgehend von der Glucose, in guten Ausbeuten hergestellt werden. Diese Verfahrensschritte sind: Glucose → 1-Amino-1-desoxy-D-glucitole → N-geschützte Verbindung → Oxidationsprodukt → I.

Demgegenüber benötigt Paulsen (loc.cit.) zur Herstellung des Desoxynojirimycins (R=H) ausgehend von der Sorbose (Glucose → Sorbit → Sorbose) neun Verfahrensstufen. Von Glucose ausgehend vermeidet also das erfindungsgemäße Verfahren acht Verfahrensschritte.

Weiterhin zeigen die vielen literaturbekannten Bemühungen (z.B. H. Paulsen und K. Todt, Adv.Carbohydr.Chem. *23*, 115 (1968); c. S. Inonye, T. Tsuroka, T. Itoh und T. Niida, Tetrahedron *23*, 2125 (1968); d. H. Saeki und E. Ohki, Chem.Pharm.Bull, *16*, 2477 (1968); e. Nippon Shinyaku, Japanische Patentanmeldungen J 55105—666 (8.2.1979) und J 55105—667 (8.2.1979)), daß die Fachwelt bis in die jüngste Zeit Desoxynojirimycin-Synthesen in Betracht gezogen hat, die vielstufig sind und eine aufwendige Schutzgruppenchemie beinhalten.

6-Amino-6-desoxy-L-sorbose liegt in saurem Medium in der Furanose-Form (XIa), im alkalischen in der Piperidinose-Form (XIb), die sich mit der Verbindung (XIc), im Gleichgewicht befindet, vor. Formel XIb ist mit Formel (III) identisch.

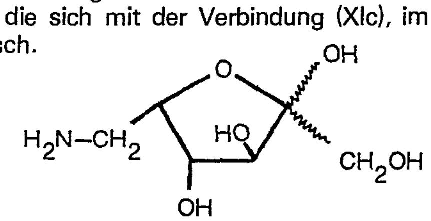

XIa

XIb 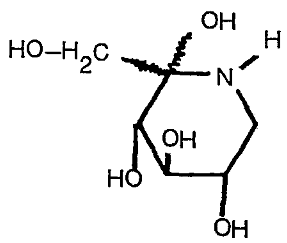 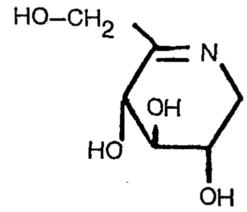 XIc

Die Verbindungen XIa, XIb und XIc sind sehr instabil und lagern sich in wäßriger Lösung, besonders im sauren pH-Bereich, durch Wasserabspaltung irreversibel in das Pyridinderivat der Formel XII um.

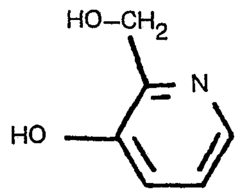 (XIII)

Diese Wasserabspaltung wird durch Substituenten der angegebenen Art am Stickstoff noch unterstützt, so daß z.B. 6-Alkylamino-6-desoxy-L-sorbosen bisher nicht bekannt geworden sind.

Es ist weiterhin bekannt (J. K. N. Jones, M. B. Perry und J. C. Turner, Can. J. Chem. *39*, 2400—2410 (1961)), daß sich 6-Desoxy-6-N-methylacetamido-L-sorbose aus 1-Desoxy-1-N-methylacetamido-D-glucitol durch 19-tägige mikrobiologische Oxidation mit Acetobacter suboxidans in 36 %iger Ausbeute herstellen läßt. Der Nachteil dieser Umsetzung ist einmal die lange Reaktionsdauer, zum anderen die Tatsache, daß die Aminogruppe durch Acetylierung geschützt werden muß. Eine Abspaltung der Acetylgruppe ohne irreversible, unerwünschte Veränderung (meist Zersetzung) ist nicht möglich.

Es ist als ausgeprochen überraschend zu bezeichnen, daß man nach dem erfindungsgemäßen mikrobiologisch/enzymatischen Verfahren die Verbindungen XI auf einfache Weise in kurzer Zeit und hoher Ausbeute erhält, und daß sich diese Verbindungen glatt und mit guter Ausbeute zu den Verbindungen der allgemeinen Formel I hydrieren lassen.

Die beim erfindungsgemäßen Verfahren eingesetzten Ausgangsverbindungen der Formel X lassen sich aus D-Glucose durch reduktive Aminierung mit entsprechenden Aminen, Wasserstoff und Nickel als Katalysator sowie nachfolgende Umsetzung mit entsprechenden Chlorameisensäureestern herstellen.

Mikroorganismen, die zur Durchführung des erfindungsgemäßen Verfahrens geeignet sind oder aus denen aktive Extrakte zur Durchführung des erfindungsgemäßen Verfahrens gewonnen werden können, können Prokaryonten, also Bakterien oder Eukaryonten, z.B. Pilze, sein und jeweils den verschiedensten taxonomischen Gruppen angehören. Der Fachmann auf mikrobiologischem Gebiet kann geeigente Mikroorganismen leicht finden, indem er eine größere Zahl aerober oder fakultativ aerober Mikroorganismen, wie sie z.B. öffentliche Sammlungen zur Verfügung stellen, in einem entsprechenden Nährmedium, das eine Verbindung der Formel X enthält, kultiviert und auf die Fähigkeit überprüft, die erfindungsgemäße Oxidationsreaktion zu katalysieren und Verbindungen der Formel XI anzuhäufen.

Indem nach dieser Vorschrift vorgegangen wurde, konnten als für das erfindungsgemäße Verfahren geeignete Mikroorganismen z.B. Bakterien der Ordnung Pseudomonadales, innerhalb dieser Ordnung besonders Vertreter der Familie Pseudomonadaceae und hier vor allen Dingen Bakterien der Gattung Gluconobacter gefunden werden. Ferner haben sich auch Bakterien aus der Gruppe der coryneformen Bakterien, besonders solche der Gattung Corynebaceterium als geeignet erwiesen. Schließlich konnte das erfindungsgemäße Verfahren auch mit Pilzen, so z.B. mit Hefen der Ordnung Endomycetales, besonders mit solchen der Familie Spermophthoraceae und hier hauptsächlich mit Vertretern der Gattung Metschnikowia durchgeführt werden.

Als Beispiele seien genannt:

Gluconobacter oxidans ssp. suboxydans (DSM 50 049), Glucobacter oxidans ssp. suboxydans (DSM 2003), Corynebacterium betae (DSM 20141) und Metschnikowia pulcherrima (ATCC 20 515).

Die DSM-Nummern geben die Nummern an, unter denen die genannten Mikroorganismen bei der Deutschen Sammlung für Mikroorganismen, Göttingen, hinterlegt sind. Mitschnikowia pulcherrima ist bei der American Type Culture Collection, Rockville Maryland USA, hinterlegt.

Wird das erfindungsgemäße Verfahren mit intakten Mikroorganismen in wachsender Kultur durchgeführt können feste, halbfeste oder flüssige Nährmedien verwandt werden. Bevorzugt werden wäßrig-flüssige Nährmedien herangezogen.

Die Kultur kann in allen Nährmedien durchgeführt werden, welche bekannterweise zur Kultivierung von Mikroorganismen der obengenannten Gruppen verwendet werden und die die im erfindungsgemäßen Verfahren zu oxidierende Verbindung der Formel X enthalten. Das Nährmedium muß assimilierbare Kohlenstoff- und Stickstoffquellen, sowie Mineralsalze enthalten. Als assimilierbare Kohlenstoff- und Stickstoffquellen eignen sich vor allem komplexe Gemische wie sie insbesondere biologische Produkte verschiedenen Ursprungs darstellen, z.B. Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt. Als Stickstoffquellen kommen zusätzlich Ammoniumsalze und Nitrate, z.B. Ammoniumchlorid, Ammoniumsulfat, Natriumnitrat und Kaliumnitrat, infrage. Die Mineralsalze, welche im Nährmedium enthalten sein sollen, liefern z.B. folgende Ionen: $Mg^{++}$, $Na^{+}$, $K^{+}$, $Ca^{++}$, $NH_4^{+}$, $Cl^{-}$, $SO_4^{--}$, $PO_4^{---}$ und $NO_3^{-}$ sowie Ionen der üblichen Spurenelemente wie Cu, Fe, Mn, Mo, Zn, Co und Ni.

Falls in den genannten komplexen Nährbodenbestandteilen oder im verwendeten Wasser diese Salze bzw. Spurenelemente nicht ausreichend vorhanden sind, ist es zweckmäßig, das Nährmedium entsprechend zu ergänzen.

Es hat sich gezeigt, daß durch Zusatz von Verbindungen des Intermediärstoffwechsels sowie von anderen Zellbausteinen zum Medium, so zum Beispiel von Aminosäuren oder von Verbindungen aus dem Tricarbonsäurezyklus eine wesentliche Verkürzung der zur vollständigen Umsetzung von Verbindungen der allgemeinen Formel (X) zu solchen der allgemeinen Formel (XI) ermöglicht wird.

Die im erfindungsgemäßen Verfahren zu oxidierende Verbindung der Formel X kann entweder allein oder im Gemisch mit einer oder mehreren oxidierbaren Verbindungen dem Grundnährmedium zugesetzt werden. Als zusätzliche oxidierbare Verbindungen können primäre Alkohole, beispielsweise Äthanol, sekundäre Alkohole, beispielsweise Isopropanol, Polyole, beispielsweise Sorbit oder Glycerin, Aldehyde, beispielsweise Glykolaldehyd, Aldoßen, beispielsweise Glucose, oder Gluconsäuren verwendet werden.

Wird der Nährlösung eine oder mehrere der genannten Verbindungen zugesetzt, kann die zu oxidierende Verbindung der Formel X entweder vor dem Animpfen oder an einem beliebigen späteren Zeitpunkt zwischen früher lag-Phase und später stationärer Wachstumsphase zugesetzt werden. In einem solchen Fall wird der betreffende Organismus auf den jeweils zugesetzten oxidierbaren Verbindungen vorkultiviert.

Für das erfindungsgemäße Verfahren ist ein pH-Bereich zwischen 2 und 10 geeignet. Es ist günstig, die Kultur in diesem Bereich zu puffern, beispielsweise mit Phosphat- oder Acetatpuffer.

Wie in der Fermentationstechnologie üblich, kann auch eine automatische pH-Regulierung durchgeführt werden, bei der sterile organische oder anorganische Säure, beispielsweise Schwefelsäure, oder sterile Lauge, beispielsweise Natronlauge, in Abständen in die Kulturlösung eingespritzt wird.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Kulturgefäße können z.B. die Dampf- als auch die Trockensterilisation angewendet werden; Luft und die Kulturmedien können ebenfalls durch Dampf, aber auch durch Filtration sterilisiert werden.

Die Beimpfung der Nährmedien erfolgt nach allgemein üblichen Methoden, z.B. über Schrägröhrchen- oder Kolbenkulturen.

Die Kultur erfolgt unter aeroben Bedingungen und kann gemäß den allgemein üblichen Methoden, beispielsweise unter Verwendung von Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern z.B. in üblichen Submersfermentationstanks. Es ist möglich, die Kultur kontinuierlich oder diskontinuierlich durchzuführen. Vorzugsweise wird diskontinuierlich gearbeitet.

Es ist zweckmäßig, sicherzustellen, daß die Mikroorganismen ausreichend mit Sauerstoff sowie den Nährstoffen in Kontakt gebracht werden. Dies kann nach der allgemein üblichen Methode wie Schütteln und Rühren, erfolgen.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxiäthylen- bzw. Polyoxipropylen-Verbindungen zugesetzt werden. Schaum kann auch mit Hilfe der üblichen mechanischen Vorrichtungen gedämpft oder beseitigt werden.

Die Züchtungstemperatur liegt zwischen 20 und 45°C. Die Dauer der Züchtung kann stark variiert werden, wobei z.B. die Zusammensetzung des Nährmediums und die Züchtungstemperatur eine Rolle spielen.

Die jeweiligen optimalen Bedingungen können von jedem Fachmann auf dem mikrobiologischen Gebiet leicht festgelegt werden.

Es hat sich herausgestellt, daß zur vollständigen Umsetzung von den der Kulturbrühe zugegebenen Verbindungen der Formel (X) in allgemeinen eine Inkubationsdauer von zwischen 3 Stunden und 7 Tragen nach Zugabe notwendig ist.

Es ist auch möglich, die erfindungsgemäße Oxidationsreaktion mit konzentrierten Zellsuspensionen geeigneter Mikroorganismen durchzuführen. Konzentrierte Zellsuspensionen werden wie folgt hergestellt: Die in Frage kommenden Mikroorganismen werden in einer geeigneten Nährlösung kultiviert, dann z.B. durch Zentrifugation geerntet und in einem kleineren Volumen derselben Nährlösung oder in Salz- oder Pufferlösungen, z.B. physiologische Kochsalzlösung, wäßrigen Lösungen von $KH_2PO_4$,

Na-Acetat, Maleinat oder einfach in Leitungs- oder destilliertem Wasser, suspendiert. Einer solchen Zellsuspension werden dann Verbindungen der allgemeinen Formel X zugesetzt und die erfindungsgemäße Oxidationsreaktion unter den oben für wachsende Kulturen beschriebenen Bedingungen durchgeführt.

Der Vorteil dieses Verfahrens ist die durch die höhere Mikroorganismen-Konzentration ermöglichte Verkürzung der Reaktionsdauer des erfindungsgemäßen Verfahrens auf wenige Stunden.

Es ist weiterhin möglich, das erfindungsgemäße Verfahren nicht nur mit wachsenden Kulturen von Mikroorganismen oder mit daraus gewonnenen konzentrierten Zellsuspensionen durchzuführen, sondern auch mit aus diesen Bakterien hergestellten Extrakten oder Extraktfraktionierungen. Dabei kann es sich um Rohextrakte handeln, wie sie durch herkömmlichen Aufschluß von Mikroorganismenzellen gewonnen werden. Als Aufschlußmethoden können dienen: Ultraschallbehandlung, Passage durch eine French-Druckzelle, Zerreiben mit Quarzsand, Inkubation mit lysierenden Enzymen, Antolyse oder mehrfaches Einfrieren und Auftauen.

Werden nichtfraktionierte Rohextrakte zur Oxidation von Verbindungen der Formel X zu Verbindungen der Formel XI verwandt, haben sich prinzipiell dieselben Reaktionsbedingungen als günstig erwiesen, wie sie für die Durchführung des erfindungsgemäßen Verfahrens mit wachsenden oder ruhenden Mikroorganismenzellen beschrieben wurden.

Soll das erfindungsgemäße Verfahren mit teilweise gereinigten Extraktpräparationen (Enzymen) durchgeführt werden, so können zur Gewinnung derartiger Präparationen die allgemein üblichen Methoden der Proteinchemie angewandt werden, wie Ultrazentrifugation, Fällungsreaktion, Ionenaustausch- oder Adsorptionschromatographie, Gelfiltration oder elektrophoretische Methoden. Zur Klärung der Frage, welche von mehreren durch eine der geannten Methoden gewonnenen Fraktionen zur Katalyse der erfindungsgemäßen Oxidationsreaktion geeignet ist, wird ein Aliquot dieser Fraktion bei einer Temperatur zwischen 20 und 45°C und einem pH zwischen 2 und 10 mit Verbindungen der Formel X gemischt und der Ansatz auf die Bildung von Verbindungen der Formel XI dünnschichtchromatographisch untersucht. Zur Durchführung der erfindungsgemäßen Reaktion mit fraktionierten Zellextrakten kann es notwendig sein, daß zusätzliche Reaktionskomponenten dem Ansatz zugefügt werden müssen, z.B. physiologische oder künstliche Elektronenakzeptoren etwa $NAD^+$, $NADP^+$, Methylenblau, Dichlorphenolindophenol, Tetrazoliumsalze etc. Müssen solche zusätzlichen Reaktionskomponenten zugefügt werden, können diese entweder in Substratmengen, also in Konzentrationen, die der der eingesetzten Verbindung der Formel X entsprechen, oder in katalytischen Mengen d.h. in Konzentrationen, die deutlich unter der gewählten Konzentration der Verbindung der Formel X liegen, eingesetzt werden.

Soll im zweiten Fall eine annähernd quantitative Durchführung des erfindungsgemäßen Verfahrens gewährleistet sein, so ist dem Reaktionsansatz weiterhin ein System zuzufügen, das die nur in katalytischer Menge vorhandene Reaktionskomponente ständig regeneriert. Es kann sich dabei z.B. um ein Enzym handeln, das für die Reoxidation eines im Verlaufe der erfindungsgemäßen Reaktion reduzierten Elektronenakzeptors in Gegenwart von Sauerstoff oder anderen Oxidationsmitteln sorgt.

Ansonsten haben sich auch für die Durchführung des erfindungsgemäßen Verfahrens mit fraktionierten Zellextrakten dieselben Bedingungen als günstig erwiesen, wie sie oben für die Oxidation von Verbindungen der Formel X zu Verbindungen der Formel XI in wachsenden Mikroorganismenkulturen oder konzentrierten Zellsuspensionen angegeben wurden. Auch hier gilt ein Temperaturbereich von 20 bis 45°C und ein pH-Bereich von 2 bis 10. Die Menge der gebildeten Verbindungen der Formel XI erreicht jedoch in einem kürzeren Zeitraum ihr Maximum. Je nach Extraktkonzentration genügt eine Inkubationszeit zwischen 2 Stunden und 3 Tagen.

Die zeitabhängige Bildung der Verbindung der Formel XI im Kulturmedium kann dünschichtchromatographisch verfolgt werden.

Die erfindungsgemäß erhaltenen Verbindungen der Formel XI werden wie folgt aus der Kulturlösung gewonnen und in die erfindungsgemäßen Verbindungen der Formel I überführt.

Die Kulturlösung wird gegebenenfalls nach Abdampfen eines Teils des Wassers im Vakuum und Abzentrifugieren oder Abfiltrieren der Zellmassen mit einem geeigneten Lösungsmittel extrahiert. Hierzu eignen sich u.a. höhere Alkohole wie Butanol, Ketone, wie Methylisobutylketon, Essigsäureeäthylester oder Gemische höherer Alkohole mit unpolaren Lösungsmitteln wie Butanol/Toluol-Gemische. Es ist auch möglich, die Kulturlösung im Vakuum zur Trockne einzudampfen und die erfindungsgemäßen Verbindungen der Formel XI aus dem Rückstand mit Alkoholen wie Methanol, Ethanol und Propanol oder den oben genannten Lösungsmitteln aufzunehmen. Aus den so erhaltenen Extrakten können die erfindungsgemäßen Verbindungen der Formel XI durch Eindampfen und gegebenenfalls nach Umkristallisation aus einem geeigneten Lösungsmittel wie Wasser oder Ethanol rein erhalten werden.

Zur Darstellung der erfindungsgemäßen Verbindungen der Formel I werden die Verbindungen der Formel XI bei einem pH-Wert zwischen 0 und 7 hydriert, wobei in den üblichen Lösungsmitteln wie Wasser, Alkoholen, Eisessig oder entsprechenden Lösungsmittelgemischen, gearbeitet wird. Durch Wahl des geeigneten Lösungsmittels oder Lösungsmittelgemisches wie Methanol/Wasser wird die gluco-Konfiguration an C—5 der erfindungsgemäßen Verbindungen der Formel I selektiv erhalten. Der geeignete pH kann mit den üblichen Mineralsäuren wie Salz oder Schwefelsäure oder mit organischen

Säuren wie Essig- oder Oxalsäure eingestellt werden. Als Katalysatoren dienen die üblichen Edelmetallkatalysatoren wie Pd, Pt oder Ni. Die gluco-Konfiguration wird insbesondere bei Hydrierung mit Pd in Wasser stereoselektiv erhalten.

Die Reaktionstemperatur kann zwischen —30 und +120°C liegen, bevorzugt wird bei Raumtemperatur gearbeitet.

Es ist ebenfalls möglich, ohne vorherige Isolierung der erfindungsgemäßen Verbindungen der Formel XI die Kulturlösungen gegebenenfalls nach Abdampfen eines Teils oder des gesamten Wassers, Abzentrifugieren oder Abfiltrieren der Zellmassen, Klären mit Aktivkohle und Ausrühren, mit einem geeigneten Edelmetall wie Raney-Nickel direkt in Analogie in der oben angegebenen Weise zu hydrieren.

Die Isolierung der erfindungsgemäßen Verbindungen der Formel I erfolgt nach Abtrennung des Katalysators durch Abdampfen des Lösungsmittels, wobei man gegebenenfalls Salze der Verbindungen der Formel I mit den bei der Hydrierung verwendeten Säuren oder der Kohlensäure erhält. Diese Salze können direkt isoliert und gegebenenfalls durch Umkristallisation gereinigt werden, oder man überführt die Salze mit einer geeigneten organischen Base wie Triethylamin oder $Ba(OH)_2$ oder besonders mit einem Anionenaustauscherharz in die freien Basen der Formel I, die gegebenenfalls durch Umkristallisation aus einem geeigneten Lösungsmittel wie Ethanol/Wasser gereinigt werden können. Zur Abtrennung größerer Mengen anorganischer Salze oder Substanzen aus dem Nährmedium aus der Kulturlösung kann es von Vorteil sein, insbesondere wenn die Verbindungen der Formel XI nicht als Reinsubstanz isoliert wurden und die Kulturlösung direkt hydriert wird, die nach der Hydrierung erhaltene Lösung über einen sauren Ionenaustauscher zu geben und die an dem Ionenaustauscher gebundenen Verbindungen der Formel I mit wäßriger oder alkoholischer Ammoniak-Lösung zu eluieren. Nach Eindampfen zur Trockene und gegebenenfalls nach Umkristallisation erhält man die reinen Verbindungen der Formel I.

Ganz besonders bevorzugt bedeuten R Wasserstoff, $C_1$-$C_{10}$-Alkyl, Hydroxyäthyl oder Arlyl und $R_1$ Benzyl oder Allyl.

## Beispiele

1. Herstellung von 1-Desoxynojirimycin durch Oxidation von 1-Benzyloxycarbonylamino-1-desoxy-D-glucitol durch Gluconobacter oxydans ssp. suboxydans in wachsender Kultur im Schüttelkolben und anschließender Hydrierung der gebildeten 6-Benzyloxycarbonylamino-6-desoxy-L-sorbose.

Gluconobacter oxidans ssp. suboxidans (DSM 50049) wurde in einer Flüssignährlösung vorkultiviert, die im Liter 20 g Hefeextrakt, 200 g Sorbit und 10 g $KH_2PO_4$ gelöst in entmineralisiertem Wasser enthielt. Die Nährlösung für die Vorkultur wurde auf einen pH-Wert von 6,2 eingestellt, zu je 100 ml in 1 l Erlenmeyerkolben gegeben und durch 15-minütiges Erhitzen im Autoklaven auf 121°C sterilisiert. Die Oxidation von Benzyloxicarbonyl-geschütztem 1-Amino-1-desoxy-D-glucitol wurde in einer Nährlösung durchgeführt, die pro Liter 20 g Hefeextrakt, 200 g Sorbit, 10 g $KH_2PO_4$ und 20 g Benzyloxycarbonyl-geschütztes 1-Amino-1-desoxy-D-glucitol gelöst in Leitungswasser enthielt. Der pH-Wert wurde auf 6,2 eingestellt. Die Nährlösung wurde zu je 100 ml in 1 l Erlenmeyerkolben gegeben undl durch 15-minütiges Erhitzen auf 121°C im Autoklaven sterilisiert. Nach dem Abkühlen wurde jeder Kolben mit jeweils 2 ml der Vorkultur beimpft und dann bei 36°C auf einer Schüttelmaschine bei 280 Umdrehungen pro Minute inkubiert. Die Bildung von Benzyloxycarbonyl-geschützter 6-Amino-6-desoxy-L-sorbose wurde dünnschicht-chromatographisch verfolgt. Nach 4 Tagen war vollständiger Umsatz erreicht. Die Kulturbrühen von 3 parallel durchgeführten Ansätzen wurden vereinigt, so daß zur Aufarbeitung ein Volumen von 300 ml zur Verfügung stand.

Die Kulturlösung wurde zentrifugiert und dreimal mit je 100 ml n-Butanol/Toluol 1:1 extrahiert. Der Extrakt wurde im Vakuum eingedampft, der Rückstand in 40 ml $H_2O$ und 60 ml Methanol aufgenommen, mit 10 ml 2 N HCL und 0,5 g Pd/C (5%) versetzt und 5 h unter Rühren hydriert. Dann wurde der Katalysator abfiltriert, die Lösung mit einem basischen Ionenaustauscher alkalisch gestellt, das Lösungsmittel im Vakuum eingedampft und der Rückstand aus Wasser/Ethanol umkristallisiert. Ausbeute: 1,1 g 1-Desoxynojirimycin (Fp. 197—199°C).

2. Glucobacter oxydans ssp. suboxydans (DSM 2003) wurde in einer Flüssignährlösung vorkultiviert, die pro 1 l 20 g Hefeextrakt, 100 g Sorbit und 10 g $KH_2PO_4$ gelöst in Leitungswasser enthielt. Die Nährlösung wurde auf einen pH-Wert von 6,2 eingestellt und im Autoklaven durch 20-minütiges Erhitzen auf 121°C keimfrei gemacht. Die Oxidation von Benzyloxycarbonyl-geschütztem 1-Amino-1-desoxy-D-glucitol wurde in einer Nährlösung mit Anfangs-pH-Wert 6,2 durchgeführt, die pro 1 l 20 g dieser Substanz sowie 20 g Hefeextrakt, 100 g Sorbit, 10 g $KH_2PO_4$, 10 g Leucin und 5 g Isoleucin enthielt. Davon wurden 250 ml in 1 l Erlenmeyerkolben gegeben und 20 Min. bei 121°C im Autoklaven behandelt. Es wurde 5 %ig mit einer gut gewachsenen Vorkultur beimpft und bei 36°C und 280 Upm auf einer Rundshüttelmaschine inkubiert. Die Bildung des Oxidationsprodukts wurde dünnschicht-chromatographisch verfolgt. Nach 3 Tagen war vollständiger Umsatz erzielt.

3 l der so erhaltenen Kulturlösung wurden im Vakuum auf 1 l eingeengt und die Lösung 3 mal durch Überschichten mit 500 ml Butanol/Toluol 10:1 und Rühren der Unterphase extrahiert. Der Extrakt wurde im Vakuum zur Trockne eingedampft und der Rückstand aus Wasser umkristallisiert. Man erhielt 51 g 6-Benzyloxycarbonylamino-6-desoxy-L-sorbose, die in 800 ml Methanol, 800 ml Wasser und 80 ml zu HCl und 2 g Pd/C (5%) unter einem Druck von 5 atm hydriert wurde. Der Katalysator wurde abgesaugt und die Lösung über einen stark sauren Ionenaustauscher (Lewatit TSW 40) gegeben, mit

Wasser nachgewaschen und mit 0,5 N $NH_3$-Lösung eluiert. Nach dem Eindampfen des Eluats wurde der Rückstand am Wasser/Ethanol umkristallisiert. Man erhielt 19 g 1-Desoxynojirimycins.

3. Herstellung von 1-Desoxynojirimycin

Der Mikroorganismus Metschnikowia pulcherrima (ATCC 20515) wurde in Schrägröhrchen auf einem Medium vorkultiviert, das pro 1 l 3 g Hefeextrakt, 6 g Pepton, 10 g Glucose, 8 g NaCl und 20 g Agar in entmineralisiertem Wasser enthielt. Mit dieser Vorkultur wurden 250 ml Flüssigmedium (in einem 1 l Erlenmeyerkolben) beimpft, das in 1 l 3 g Hefeextrakt, 6 g Pepton, 10 g Sorbit, 8 g NaCl und 10 g Benzyloxycarbonylgeschütztes 1-Amino-1-desoxy-D-glucitol gelöst in entmineralisiertem Wasser enthielt und im Autoklaven durch Erhitzen für 20 Minuten auf 121 °C sterilisiert worden war. Die Kultur wurde bei 35°C auf einer Rundschüttelmaschine bei 200 Upm inkubiert. Der Gehalt an Benzyloxycarbonyl-geschützter 6-Amino-6-desoxy-L-sorbose in der Kulturbrühe wurde dünnschichtchromatographisch bestimmt. Nach 2 Tagen waren 4 g/l (40%) umgesetzt. Die Fermentation wurde zu diesem Zeitpunkt abgebrochen, die Kulturlösung wie in Beispiel 2 angegeben aufgearbeitet. Die 6-Benzyloxycarbonylamino-6-desoxy-L-sorbose konnte durch Umkristallisieren aus Wasser und aus Ethanol rein erhalten und wie in Beispiel 1 angegeben zu 1-Desoxynojirimycin hydriert werden.

4. Herstellung von 1-Desoxynojirimycin.

Der Mikroorganismus Corynebacterium betae (DSM 20141) wurde auf Schrägröhrchen vorkultiviert, die folgende Nährböden enthielten 10 g/l tryptisch verdautes Casein-Pepton, 6 g/l Hefeextrakt, 5 g/l Sorbit, 5 g/l NaCl und 20 g/l Agar in entmineralisiertem Wasser. Mit einem gut bewachsenen Schrägröhrchen wurden 250 ml Flüssignährlösung (im 1 l Erlenmeyerkolben) beimpft, die pro 1 l 10 g tryptisch verdautes Casein-Pepton, 5 g Hefeextrakt, 5 g Sorbit, 5 g NaCl und 10 g Benzyloxycarbonylgeschütztes 1-Amino-1-desoxy-D-glucitol enthielt. Die Nährbodenbestandteile wurden in entmineralisiertem Wasser gelöst und durch 20-minütiges Erhitzen im Autoklaven auf 121 °C sterilisiert. Die Kultur wurde bei 37°C auf einer Rundschüttelmaschine bei 200 Upm inkubiert. Der Gehalt an Benzyloxycarbonyl-geschützter 6-Amino-6-desoxy-L-sorbose wurde dünnschicht-chromatographisch bestimmt. Nach 2 Tagen waren 3 g/l (entsprechend 30%) umgesetzt. Zu diesem Zeitpunkt wurde die Fermentation abgebrochen, die Zellen durch Zentrifugation abgetrennt und der klare Kulturüberstand wie in Beispiel 3 angegeben aufgearbeitet.

5. Herstellung von 1-Desoxynojirimycin durch Oxidation von Benzyloxycarbonyl-geschütztem 1-Amino-1-desoxy-D-glucitol durch Glucobacter oxydans ssp. suboxydans in wachsender Kultur im Fermenter und anschließender Hydrierung der gebildeten Benzyloxycarbonyl-geschützten 6-Amino-6-desoxy-L-sorbose.

Glucobacter oxydans ssp. suboxydans (DSM 2003) wurde auf Schrägröhrchen vorkultiviert. Der Nährboden enthielt pro 1 l 10 g Hefeextrakt, 100 g Sorbit und 2 g $KH_2PO_4$, gelöst in Leitungswasser. Mit einem gut bewachsenen Röhrchen wurde eine Flüssigkultur von 250 ml desselben Mediums jedoch ohne Agar in einem 1 l Erlenmeyerkolben beimpft und bei 36°C auf einer Rundschüttelmaschine bei 280 Upm über Nacht inkubiert. Mit dieser 2. Vorkultur wurde ein 10 l Fermenter beimpft, der mit einem Flüssigmedium beschicht war, das pro 1 l 10 g Hefeextrakt, 100 g Sorbit, 2 g $KH_2PO_4$ und 10 g Benzyloxycarbonyl-geschütztes 1-Amino-1-desoxy-D-glucitol enthielt. Der pH-Wert war auf 6,2 eingestellt worden. Das Medium war durch 15-minütiges Erhitzen im Autoklaven auf 121 °C sterilisiert worden. Der Fermenter wurde mit 5 l Luft pro Minute durchblasen und mit 500 Upm gerührt. Die Inkubationstemperatur war 36°C. Zu verschiedenen Zeiten wurden Proben steril entnommen und der Gehalt an Benzyloxycarbonyl-geschützter 6-Amino-6-desoxy-L-sorbose dünnschicht-chromatographisch bestimmt. Nach 2 1/2 Tagen war vollständige Umsetzung erzielt.

Die Kulturlösung wurde im Vakuum auf 2 l eingeengt, der ausgefallene Niederschlag durch Erwärmen auf 60—80°C wieder in Lösung gebracht, die trübe Lösung mit Aktivkohle gerührt und filtriert. Aus dem Filtrat fiel 6-Benzoyloxycarbonylamine-6-desoxy-L-sorbose aus, die einmal aus Wasser und einmal aus Ethanol umkristallisiert wurde. Ausbeute 90 g. Das Produkt wurde in der üblichen Weise zu 1-Desoxynojirimycin hydriert.

6. Herstellung von 1-Desoxynojirimycin.

Glucobacter oxydans ssp. suboxydans (DSM 2003) wurde im 10 l Maßstab im Fermenter in einem Medium (Anfangs-pH-wert: 6,2), das pro 1 l 100 g Sorbit, 20 g Hefeextrakt und 2 g $KH_2PO_4$ — gelöst in Leitungswasser — enthielt. Der Fermenter wurde mit 5 l Luft pro Minute durchblasen, mit 500 Upm gerührt und bei 36°C temperiert. Nach 10-stündiger Inkubation wurden die Zellen aus der Kulturbrühe abzentrifugiert und in 1 l eines Mediums suspendiert, das 20 g Hefeextrakt, 2 g $KH_2PO_4$ und 20 g Benzyloxycarbonyl-geschütztes Amino-1-desoxy-D-glucitol enthielt. Diese 10-fach konzentrierte Zellsuspension wurde in einem 1 l Fermenter bei 36°C inkubiert, mit 5 l Luft pro Minute durchblasen und mit 500 Upm gerührt. Nach 24 Stunden war vollständige Umsetzung erreicht. Die Zellen wurden abzentrifugiert und der Überstand, wie in Beispiel 1 angegeben aufgearbeitet und zu 1-Desoxynojirimycin hydriert.

7. Herstellung von 1-Desoxynojirimycin.

Glucobacter oxydans ssp. suboxydans (DSM 2003) wurde im 10 l Maßstab im Fermenter in einem Medium vorkultiviert, das pro 1 l 100 g Sorbit, 20 g Hefeextrakt und 2 g $KH_2PO_4$ — gelöst in Leitungswasser — enthielt. Der Fermenter wurde mit 5 l Luft pro Minute durchblasen, mit 500 Upm gerührt und bei 36°C temperiert. Nach 16-stündiger Inkubation wurden die Zellen aus der Kulturbrühe

abzentrifugiert, durch Suspension in 10mM $KH_2PO_4$ und erneutem Zentrifugieren einmal gewaschen und dann in 300 ml 10 mM $KH_2PO_4$ suspendiert. Die so erhaltene Zellsuspension wurde durch zweimalige Passage durch eine French-Druckzelle bei 8 bar aufgeschlossen. Der zellfreie Extrakt wurde in einen 1 l Erlenmeyerkolben gegeben, mit Benzyloxycarbonylgeschützter 1-Amino-1-desoxy-D-glucitol zu einer Konzentration von 20 g/l versetzt und bei 36°C auf einer Rundschüttelmaschine bei 280 Upm inkubiert.

Nach 17 Stunden war vollkommene Umsetzung erreicht. Die Kulturlösung (300 ml) wurde wie in Beispiel 1 aufgearbeitet. Nach Hydrierung erhielt man 1,8 g 1-Desoxynojirimycin.

8. Herstellung von 1-Desoxynojirimycin durch Oxidation von Benzyloxycarbonyl-geschütztem 1-Amino-1-desoxy-D-glucitol in einer definierten Enzymreaktion in einem zellfreien Extrakt von Glucobacter oxydans ssp. suboxydans und anschließender Hydrierung der gebildeten Benzyloxycarbonyl-geschützten 6-Amino-6-desoxy-L-sorbose.

Ein zellfreier Extrakt von Glucobacter oxydans ssp. suboxydans wurde wie in Beispiel 6 angegeben hergestellt. Dem Extrakt wurde zugegeben:

Niconsäureamid-adenin-dinucleotidphosphat als Na-Salz (NADP) zu einer Endkonzentration von 0,2 mM, eine mikrosomale Rohfraktion der Hefe Saccharomyces cerevisiae (ATCC 287) (1 mg/ml) als NADP-regenerieren-des System und 1-Benzyloxy-carbonyl-geschütztes 1-Amino-1-desoxy-D-glucitol (10 g/l).

Das Gemisch wurde bei 36°C im Wasserbad inkubiert und mit Sauerstoff durchblasen. Die Bildung Benzyloxycarbonyl-geschützter 6-Amino-desoxy-L-sorbose wurde dünnschicht-chromatographisch gemessen. Nach 13 Stunden war ein Gehalt von 7 g 6-Amino-6-desoxy-L-sorbose pro 1 l nachweisbar — entsprechend einer 70 %igen Umsetzung —. Zu diesem Zeitpunkt wurde die Reaktion abgebrochen und das Gemisch wie in Beispiel 3 angegeben aufgearbeitet und die erhaltene 6-Benzyloxycarbonylamino-6-desoxy-L-sorbose zu 1-Desoxynojirimycin hydriert.

9. Herstellung von 1-Desoxy-N-methylnojirimycin durch Oxidation von Benzyloxycarbonyl-geschütztem N-Methyl-1-amino-1-desoxy-D-glucitol durch Glucobacter oxydans ssp. suboxydans in wachsender Kultur im Schüttelkolben und anschließender Hydrierung der gebildeten Benzyloxy-carbonyl-geschützten N-Methyl-6-amino-6-desoxy-L-sorbose.

Glucobacter oxydans ssp. suboxydans wurde wie in Beispiel 2 beschrieben vorkultiviert. Die Oxidationsreaktion wurde wie dort beschrieben durchgeführt, jedoch wurde statt des Benzyloxy-carbonyl-geschütztem 1-Amino-1-desoxy-D-glucitols die entsprechende N-Methylverbindung zu einer Konzentration von 20 g/l zugesetzt. Nach 4 Tagen war eine 100 %ige Umsetzung erzielt.

300 ml der Kulturlösung wurden aufgearbeitet, in dem die Zellmassen abzentrifugiert und die klare Lösung 3 x mit je 100 ml n-Butanol/Toluol 10:2 extrahiert, der Extrakt einrotiert und der Rückstand 2 x aus Wasser umkristallisiert wurde. Man erhielt 5,5 g 6-Desoxy-N-benzyloxycarbonyl-6-methylamino-L-sorbose, die unter den üblichen Bedingungen zu 1-Desoxy-N-methylnojirimycin hydriert wurde. Man erhielt 2,1 g 1-Desoxy-N-methylnojirimycin vom Schmelzpunkt 152°C (Ethanol).

## Beispiel 10

Herstellung von 1-Desoxynojirimycin durch Oxidation von 1-Benzyloxycarbonylamino-1-desoxy-D-glucitol durch Gluconobacter oxydans ssp. suboxydans im Schüttelkolben und anschließender Hydrierung der gebildeten 6-Benzyloxycarbonylamino-6-desoxy-1-sorbose.

Gluconobacter oxidans ssp. suboxidans (DSM 50049) wurde in einer Flüssignährlösung vorkultiviert, die im Liter 20 g Hefeextrakt, 200 g Sorbit und 10 g $KH_2PO_4$ gelöst in entmineralisiertem Wasser enthielt. Die Nährlösung wurde auf einen pH-Wert von 6,2 eingestellt, zu je 250 ml in 1 l Erlenmeyerkolben gegeben, durch 15-minütiges Erhitzen im Autoklaven auf 121°C sterilisiert und nach dem Abkühlen 2 %-ig mit einer im selben Medium gezüchteten Vorkultur beimpft. Die Bebrütung erfolgte auf einer Rundschüttelmaschine bei 28°C und 280 UPm.

Nach 36 Stunden wurden in jeden Kolben 25 ml einer 70—90°C heißen 20 %-igen Lösung von Benzyloxicarbonyl-geschütztem 1-Amino-1-desoxy-D-glucitol in destilliertem Wasser gegeben. Diese Lösung war durch vorheriges Erhitzen im Autoklaven (5' bei 105°C) keimfrei gemacht worden. Die Kolben wurden dann weiter bei 28°C und 280 Upm inkubiert: Die Bildung von Benzyloxycarbonyl-geschützter 6-Amino-6-desoxy-L-sorbose wurde dünnschicht-chromatographisch verfolgt. Nach 2 Tagen war vollständiger Umsatz erreicht. Zu diesem Zeitpunkt und nach weiteren 2 Tagen wurde die Zufütterung von Benzyloxicarbonyl-geschütztem 1-Amino-1-desoxy-D-glucitol in gleicher Weise zweimal wiederholt. Nach 8 Tagen war vollständiger Umsatz erreicht. Die Kulturbrühen von 3 parallel durchgeführten Ansätzen wurden vereinigt, so daß zur Aufarbeitung ein Volumen von 750 ml zur Verfügung stand.

Die Kulturlösung wurde 3 Tage bei 0 bis 5°C aufbewahrt, der auskristallisierte Niederschlag abgetrennt, in 500 ml Methanol gelöst, die unlöslichen Zellrückstände abzentrifugiert und die klare Lösung eingedampft. Das erhaltene kristalline Produkt wurde einmal aus Isopropanol umgelöst. Man erhielt 40 g 6-Benzyloxycarbonylamino-6-desoxy-L-sorbose (Fp. 107—111°C). Zur Überführung in 1-Desoxynojirimycin wurde die Substanz in 500 ml Methanol gelöst, zu 0,4 g $K_2CO_3$ und 10 g 5% Pd/c in 1 $H_2O$ gegeben und 3 h bei 80 atm $H_2$ und 50—60°C hydriert. Nach dem Absaugen des Katalysators

wurde der Ansatz im Vakuum zur Trockne eingedampft und der Rückstand aus Methanol umkristallisiert.

Ausbeute 16 g, Fp. 202—205°C.

Beispiel 11

Herstellung von 1-Desoxynojirimycin durch Oxidation von 1-Alkyloxycarbonylamino-1-desoxy-D-glucitol durch Glucobacter oxydans ssp. suboxydans im Schüttelkolben und anschließender Hydrierung der 6-Alkyloxycarbonylamino-6-desoxy-L-sorbose.

Glucobacter oxydans ssp. suboxydans (DSM 2003) wurde wie in Beispiel 10 beschrieben in 250 ml einer Nährlösung, die Hefeextrakt und Sorbit enthielt, vorkultiviert. Nach 2 Tagen wurden 25 ml einer 10 %-igen Lösung von allyl-geschütztem 1-Amino-1-desoxy-D-glucitol zugegeben, die zuvor durch Erhitzen im Autoklaven (5 Min. 105°C) sterilisiert worden war. Die Umsetzung wurde durch Dünnschichtchromatographie verfolgt. Sie war nach 36 Stunden quantitativ. Zu diesem Zeitpunkt wurde die Fermentation abgebrochen, die Kulturlösung im Vakuum auf 100 ml eingeengt, 3 mal mit je 50 ml Butanol extrahiert, der Extrakt eingedampft, der Rückstand von 200 g Kieselgel mit Essigester/Methanol/-Wasser 10:3:2 chromatographiert. Nach dem Umkristallisieren aus Acetonnitrit wurden 1,3 g 6-Allyloxycarbonylamino-6-desoxy-L-sorbose erhalten, die in 10 ml Wasser gelöst und bei 50—60°C und 80 atm in Gegenwart von 1 g 10 %-igem Pd auf Kohle 8 h hydriert wurde. Nach dem Abfiltrieren des Katalysators, Eindampfen der Lösung und Umkristallisation aus Methanol erhielt man 0,7 g 1-Desoxynojirimycin.

Beispiel 12

Herstellung von 1-Desoxy-N-methylnojirimycin durch Oxidation von 1-Benzyloxycarbonylamino-1-desoxy-N-methyl-D-glucitol durch Glucobacter oxydans ssp. suboxydans im Schüttelkolben und anschließender Hydrierung der gebildeten 6-Benzyloxycarbonyl-amino-6-desoxy-N-methyl-L-sorbose.

Glucobacter oxydans ssp. suboxydans (DSM 2003) wurde wie in Beispiel 10 beschrieben in 250 ml einer Nährlösung, die Hefeextrakt und Sorbit enthielt, vorkultiviert. Nach 2 Tagen wurden 25 ml einer 70—90°C heißen 10 %-igen Lösung von 2,5 g 1-Benzyloxycarbonylamino-1-desoxy-N-methyl-D-glucitol in destilliertem Wasser zugegeben, die zuvor durch Erhitzen im Autoklaven (5 Min. 105°C) sterilisiert worden war. Die Umsetzung wurde durch Dünnschichtchromatographie verfolgt. Sie war nach 36 Stunden quantitativ. Zu diesem Zeitpunkt wurde die Fermentation abgebrochen. 10 Kolben mit einem Gesamtvolumen von 2,5 l wurden zusammengegeben, das Vakuum auf 0,5 l eingeengt und 3 Tage bei 0 bis 5°C aufbewahrt. Der auskristallisierte Niederschlag wurde abgesaugt und 2 mal aus Wasser umkristallisiert. Es wurden 13 g 6-Benzyloxycarbonylamino-l-desoxy-N-methyl-L-sorbose vom Fp. 105—107°C erhalten. Zur Überführung in 1-Desoxy-N-methylnojirimycin wurde die Substanz in 150 ml Methanol gelöst, 300 ml $H_2O$, 0,4 ml 10 %-ige $K_2CO_3$-Lösung und 5 g 5 %-iges Pd auf Kohle hinzugegeben und 3 h bei 80 atm und Raumtemperatur hydriert. Nach dem Absaugen des Katalysators wurde der Ansatz eingedampft und der Rückstand aus Ethanol/Wasser umkristallisiert.

Ausbeute: 6 g 1-Desoxy-N-methylnojirimycin, Fp. 151—3°C.

Beispiel 13

Herstellung von 1-Desoxy-N-hydroxyethylnojirimycin durch Oxidation von Benzyloxycarbonyl-geschütztem 1-(2-Hydroxyethylamino)-1-desoxy-D-glucitol durch Glucobacter oxydans ssp. suboxydans im Fermenter und anschließender Hydrierung der gebildeten Benzyloxycarbonyl-geschützten 1-(2-Hydroxyethylamino)-6-desoxy-L-sorbose.

Glucobacter oxydans ssp. suboxydans wurde im 10 l Fermenter in einem Medium, das 5% Sorbit, 2% Hefeextrakt, 0,4% $KH_2PO_4$ und 0,04% Polyolentschäumer enthielt und 45 Minuten bei 121°C im Autoklaven keimfrei gemacht worden war, vorkultiviert. Nach 24 Std. wurden 500 ml einer 20 %-igen Lösung von Benzyloxycarbonyl-geschütztem 1-(2-Hydroxyethylamino)-1-desoxy-D-glucitol im destilliertem Wasser der Kultur zugefüttern. Die Lösung war durch Sterilfiltration keimfrei gemacht worden. Das Zufüttern wurde am 2. und am 3. Tag wiederholt. Am 4. Tag war das gesamte Substrat umgesetzt. Die Fermentation wurde zu diesem Zeitpunkt abgebrochen, die Kulturlösung im Vakuum zur Trockne eingedampft, der Rückstand 3 mal mit je 1 l Ethanol ausgerührt, die Ethanol-Phase eingedampft und der Rückstand an 1 kg Kieselgel mit Essigester/Methanol/Wasser 10:3:2 gereinigt. Die erhaltene 6-Benzyloxycarbonyl-6-desoxy-N-(2-hydroxyethyl)-L-sorbose (180 g) wurden analog Beispiel 10 hydriert und das erhaltene 1-Desoxy-N-hydroxyethylnojirimycin aus Ethanol/Wasser oder Methylglycol umkristallisiert.

Ausbeute: 70 g, Fp. 141—3°C.

Beispiel 14

Herstellung von 1-Benzyloxycarbonylamino-1-desoxy-D-glucitol:

Zu 3.7 kg 1-Amino-1-desoxy-D-glucitol (etwa 70 %-ig) in 7 l $H_2O$ tropfte unter kräftigem Rühren Benzyloxycarbonylchlorid und hielt den pH mit 2 N Natronlauge bei 8—9. Hierbei ließ man den Ansatz auf 40—50°C erwärmen. Anschließend wurde über Nacht bei Raumtemperatur nachgerührt, der Niederschlag abgesaugt, mit Aceton gewaschen und aus Wasser umkristallisiert.

Ausbeute 3 kg, Fp. 143—5°C.

Beispiel 15

Herstellung von 1-Benzyloxycarbonylamino-1-desoxy-N-methyl-D-glucitol:

Analog Beispiel 16 aus 1-Methylamino-1-desoxy-D-glucitol, umkristallisiert aus Ethanol. Fp. 123—5°C.

Beispiel 16

Herstellung von 1-Allyloxycarbonylamino-1-desoxy-D-glucitol und Allyloxycarbonylchlorid. Zur Aufarbeitung wurde der Ansatz mit Essigester extrahiert, über einen Mischbett-Ionenaustauscher entsalzt, eingedampft und der Rückstand aus Ethanol umkristallisiert.

Fp. 99—102°C.

Beispiel 17

Herstellung von 1-Benzyloxycarbonylamino-1-desoxy-N-(2-hydroxyethyl)-D-glucitol. Analog Beispiel 16, schlecht umkristallisierende Verbindung.

**Patentansprüche**

1. Verfahren zur Herstellung der Verbindungen der Formel

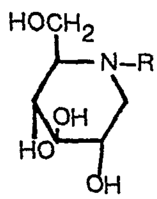

(I)

worin

R Wasserstoff, gegebenenfalls durch OH, $C_1$-$C_4$-Alkoxy oder Di-$C_1$-$C_4$-alkylamino substituiertes $C_1$-$C_{10}$-Alkyl bedeutet, dadurch gekennzeichnet, daß man

a) Verbindungen der allgemeinen Formel

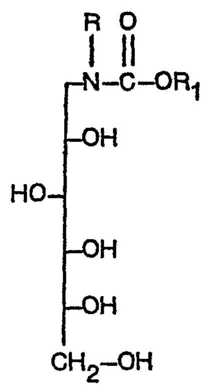

worin

R die bereits genannte Bedeutung hat und

$R_1$ gegebenenfalls durch Chlor, Brom, Nitro, Methyl oder Methoxy substituiertes Benzyl oder Allyl bedeutet,

in an sich bekannter Weise mit aeroben Mikroorganismen oder Extrakten daraus in geeigneten Medien, die assimilierbare Kohlenstoff- und Stickstoffquellen sowie Mineralsalze enthalten und bei pH-Werten zwischen 2 und 10 und bei Temperaturen zwischen 20 und 45°C zu Verbindungen der Formel

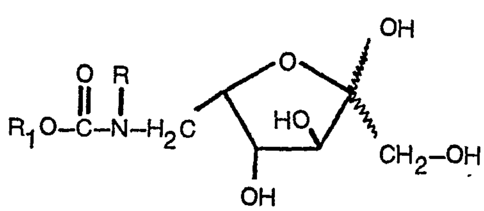

worin R und $R_1$ die bereits genannte Bedeutung haben, umsetzt wobei als aerobe Mikroorganismen solche eingesetzt werden, die in einem entsprechenden Nährmedium enthaltend eine Verbindung der Formel

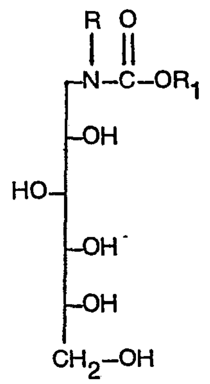

worin R und $R_1$ die angegebene Bedeutung besitzen, eine Verbindung der Formel

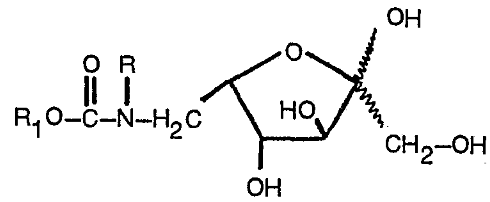

anhäufen, und

b) diese Verbindungen bei einem pH-Wert zwischen 0 und 7 und in einem geeigneten Lösungsmittel unter Erhalt der Konfiguration katalytisch hydriert.

2. Verfahren nach Anspruch 1, worin R Wasserstoff, $C_1$-$C_{10}$-Alkyl oder Hydroxyäthyl und $R_1$ Benzyl oder Allyl bedeuten.

3. Verfahren nach Anspruch 1 oder 2, dadurch gekennzeichnet, daß man als Mikroorganismen Bakterien der Ordnung Pseudomonadales, coryneforme Bakterien oder Hefen der Ordnung Endomycetales einsetzt.

4. Verfahren nach den Ansprüchen 1—3, dadurch gekennzeichnet, daß man als Mikroorganismen Gluconobacter oxidans ssp. suboxydans (DSM 50 049), Glucobacter oxidans ssp. suboxydans (DSM 2003), Corynebacterium betae (DSM 20141) oder Metschnikowia pulcherrima (ATCC 20 515) einsetzt.

**Revendications**

1. Procédé de production des composés de formule:

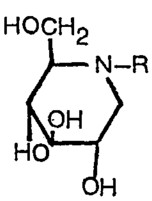

dans laquelle:

R est l'hydrogène, ou un reste alkyle en $C_1$ à $C_{10}$ éventuellement substitué par OH, un reste alkoxy en $C_1$ à $C_4$ ou un reste di-(alkyle en $C_1$ à $C_4$)amino, caractérisé en ce que

a) on transforme des composés de formule générale:

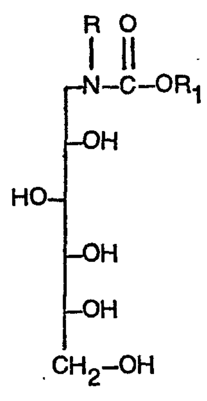

dans laquelle:

R a la définition déjà donnée et

$R_1$ est un reste benzyle ou allyle éventuellement substitué par du chlore, du brome ou un radical nitro, méthyle ou méthoxy, d'une manière connue avec des micro-organismes aérobies ou des extraits de ces micro-organismes dans des milieux convenables qui contiennent des sources de carbone et d'azote assimilables ainsi que des sels minéraux et à des valeurs de pH comprises entre 2 et 10 et à des températures comprises entre 20 et 45°C, en composés de formule:

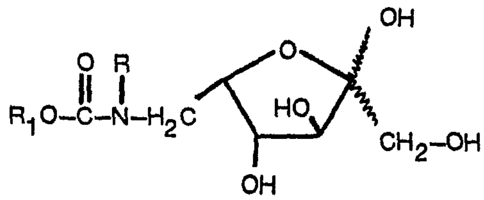

dans laquelle R et $R_1$ ont la définition déjà donnée, en utilisant comme micro-organismes aérobies des micro-organismes qui accumulent, dans un milieu nutritif correspondant contenant un composé de formule:

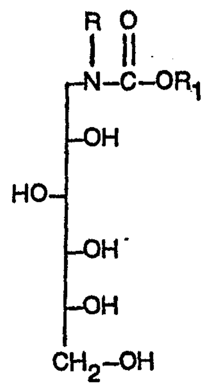

dans laquelle R et $R_1$ ont la définition déjà indiquée, un composé de formule:

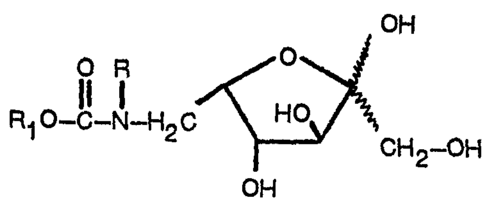

et

b) on hydrogène catalytiquement ces composés à un pH d'une valeur de 0 à 7 et dans un solvant convenable en maintenant la configuration.

2. Procédé suivant la revendication 1, dans lequel R est l'hydrogène, un reste alkyle en $C_1$ à $C_{10}$ ou un reste hydroxyéthyle et $R_1$ est un reste benzyle ou allyle.

3. Procédé suivant la revendication 1 ou 2, caractérisé en ce qu'on utilise comme micro-organismes des bactéries de l'ordre des pseudomonadales, des bactéries corynéformes ou des levures de l'ordre des endomycétales.

4. Procédé suivant les revendications 1 à 3, caractérisé en ce qu'on utilise comme micro-organismes Gluconobacter oxidans ssp. suboxydans (DSM 50 049), Glucobacter oxidans ssp. suboxydans (DSM 2003), Corynebacterium betae (DSM 20141) ou Metschinikowia pulcherrima (ATCC 20 515).

## Claims

1. Process for the preparation of compounds of the formula

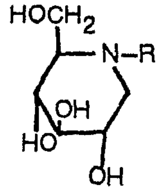

wherein

R denotes hydrogen, $C_1$-$C_{10}$-alkyl which is optionally substituted by OH, $C_1$-$C_4$-alkoxy or di-$C_1$-$C_4$-alkylamino, characterised in that

a) compounds of the general formula

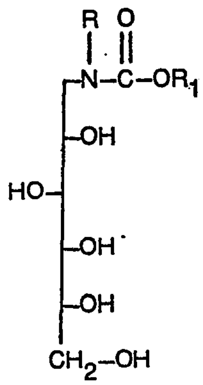

wherein

R has the meaning already mentioned and

$R_1$ denotes benzyl which is optionaly substituted by chlorine, bromine, nitro, methyl or methoxy, or allyl, are reacted, in a manner known per se, with aerobic microorganisms or extracts thereof in suitable media which contain sources of carbon and nitrogen which can be assimilated, and mineral salts, and at pH values between 2 and 10 and at temperatures between 20 and 45°C to give compounds of the formula

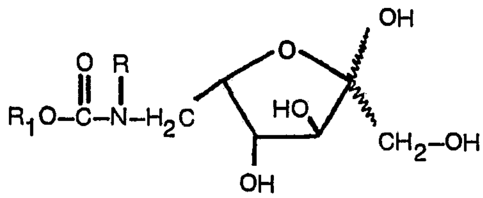

wherein

R and $R_1$ have the meaning already mentioned, the aerobic micro-organisms used being those which, in a corresponding nutrient medium containing a compound of the formula

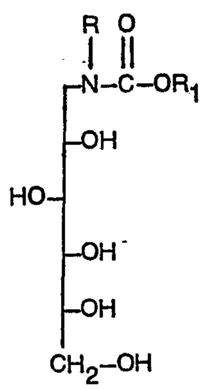

wherein

R and $R_1$ have the indicated meaning, accumulate a compound of the formula

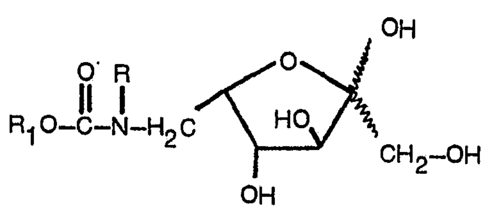

and

b) these compounds are subjected to catalytic hydrogenation at a pH value between 0 and 7 and in a suitable solvent with retention of the configuration.

2. Process according to Claim 1, wherein
R denotes hydrogen, $C_1$-$C_{10}$-alkyl or hydroxyethyl and
$R_1$ denotes benzyl or allyl.

3. Process according to Claim 1 or 2, characterised in that the micro-organisms employed are bacteria of the order Pseudomonadales, coryneform bacteria or yeasts of the order Endomycetales.

4. Process according to Claims 1 to 3, characterised in that the micro-organisms employed are Gluconobacter oxidans ssp. suboxydans (DSM 50,049), Glucobacter oxidans ssp. suboxydans (DSM 2003), Corynebacterium betae (DSM 20141) or Metschnikowia pulcherrima (ATCC 20,515).